# NOUVEAU FASCICULE DE BREVET EUROPEEN

(11) **EP 0 970 682 B2**
(45) Date de publication et mention de la décision concernant l'opposition: **26.01.2005**
(45) Mention de la délivrance du brevet: 11.04.2001
(21) Numéro de dépôt: 99401598.0
(22) Date de dépôt: 25.06.1999
(51) Int. Cl.: A61K 7/00

(54) **Composition sous forme d'émulsion eau-dans-huile ayant une vitesse de cisaillement évolutive**
Wasser-in-Öl Zusammensetzung mit verlaufsveränderlicher Schergeschwindigkeit
Water-in-oil composition with a variable shear rate

(30) Priorité: 01.07.1998 FR 9808418
(43) Date de publication de la demande: 12.01.2000
(73) Titulaire: L'OREAL, 75008 Paris (FR)
(72) Inventeur: Afriat, Isabelle, 75003 Paris (FR); Boulier, Virginie, 91550 Paray Vieille-Poste (FR)
(74) Mandataire: Rasson, Catherine

(56) Documents cités:
- EP-A- 0 334 486
- EP-A- 0 407 089
- EP-A- 0 490 582
- EP-A- 0 529 847
- EP-A- 0 612 517
- EP-A- 0 768 080
- WO-A-95/15812
- WO-A-96/36323
- FR-A- 2 686 510
- Extrait du CTFA On-Line, Dow Corning 3225C
- Cyclomethiconhaltige kosmetische W/O-Lotionen auf Basis siliciumorganischer Emulgatoren; Peter Hameyer: Seifen-Oele-Fette-Wachse Nr. 10/1990, pp. 392-395
- Brochure "A Guide to Formulating Water-in-Silicone Emulsions with DOW CORNING 3225C Formulation Aid" de Dow Corning, 1996
- Brochure "Information About Volatile Silicone Fluids de DOW CORNING" de Dow Corning, 1993
- Brochure "Dimethicone Copolyols for Cosmetic and Toiletry Application" de Dow Corning, 1998
- International Cosmetic Ingredient Dictionary and Handbook, 7th edition 1997, pp. 432,433
- CTFA Cosmetic Ingredient Dictionary, 3rd edition, 1982, pp. 60, 83, 84
- Brochure "Information About Dow Corning 3225 Formulation Aid" de Dow Corning, 1997
- Brochure "A Guide to Formulating Water-in-Silicone Emulsions with DOW CORNING 3225C Formulation Aid" de Dow Corning

## Description

L'invention se rapporte à une composition se présentant sous forme d'une émulsion eau-dans-huile (E/H) ayant une viscosité à un taux de cisaillement de 200 s⁻¹ et à 25 °C, allant de 3 Pa.s (30 poises) à 20 Pa.s (200 poises) et comportant une forte teneur en eau et un tensioactif siliconé particulier. Cette composition a l'aspect d'une crème et est utilisable en particulier dans les domaines cosmétique et/ou dermatologique.

Dans les domaines cosmétique ou dermatologique, il est courant d'utiliser des compositions ayant l'aspect d'une crème et constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Une crème est, dans les domaines considérés, une composition présentant une certaine viscosité, par opposition aux compositions liquides ou semi-liquides telles que les lotions et les laits, ou encore aux compositions solides.

Toutefois, les crèmes sous forme d'émulsions E/H présentent l'inconvénient d'apporter sur la peau à l'application, un toucher assez gras, la phase huileuse étant la phase externe. Ainsi, ces crèmes sont en général utilisées pour les peaux sèches, étant trop grasses pour être utilisées sur les peaux grasses. De plus, les émulsions E/H n'apportent aucune fraicheur et sont généralement trop riches en huiles pour être utilisées pendant l'été ou dans les pays chauds.

Le document EP-612517 décrit une émulsion E/H comprenant comme émulsionnant un alkyldiméthicone copolyol, un alcoxydiméthicone copolyol, ou un diméthicone copolyol exempt de groupe oxypropyléné.

Pour surmonter ces inconvénients, il a été envisagé de préparer des émulsions à forte teneur en eau. Toutefois, la teneur en eau ne peut pas être trop importante pour des raisons de stabilité, ou alors une forte teneur en eau doit être compensée par l'ajout de plusieurs tensioactifs ou d'agents gélifiants qui peuvent nuire au confort de la composition finale et même entraîner des problèmes d'irritations cutanés notamment chez les sujets à peaux sensibles.

Il subsiste donc le besoin d'une composition ayant la viscosité d'une crème et se présentant sous forme d'une émulsion eau-dans-huile stable, comportant une quantité importante d'eau et utilisable dans les domaines cosmétique et/ou dermatologique, qui ne présente pas les inconvénients de l'art antérieur.

La demanderesse a maintenant trouvé une composition du type émulsion eau dans huile permettant d'atteindre ces objectifs.

Cette composition comprend une phase aqueuse dispersée dans une phase huileuse à l'aide d'un agent émulsionnant siliconé, caractérisée par le fait qu'elle a une viscosité mesurée au viscosimètre RHEOMAT 180 à un taux de cisaillement de 200 s⁻¹ et à 25 °C, allant de 3 Pa.s (30 poises) à 20 Pa.s (200 poises), qu'elle comporte au moins 78 % en poids de phase aqueuse par rapport au poids total de la composition dont au moins 65 % d'eau par rapport au poids total de la composition, et qu'elle contient comme seul agent émulsionnant, le diméthicone copolyol de formule (I) : 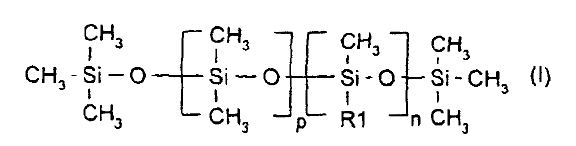 dans laquelle R1 représente -(C₃H₆O)-(OC₂H₄)₁₈-(C₃H₆O)₁₈H, p=394 et n=4,

En dépit de la quantité importante d'eau, la composition de l'invention est stable dans le temps. En outre, elle possède une caractéristique rhéologique spécifique qui rend son utilisation dans les domaines considérés, particulièrement intéressante. En effet, lors de l'application sur la peau, elle "casse", c'est-à-dire qu'elle se fluidifie brutalement sous l'effet du cisaillement, ce qui est probablement dû à un phénomène de rupture au sein de l'émulsion. Ainsi, la composition de l'invention apporte une très grande fraîcheur sur la peau.

La composition selon l'invention a une viscosité allant de 3 Pa.s (30 poises) à 20 Pa.s (200 poises). Cette viscosité est mesurée au Rhéomat 180, c'est-à-dire avec l'appareil RM180 Rheomat de la société METTLER.

La composition selon l'invention comporte au moins 78 % en poids de phase aqueuse par rapport au poids total de la composition et de préférence au moins 80 % du poids total de la composition. La phase aqueuse peut constituer jusqu'à 92 % du poids total de la composition.

L'eau constitue au moins 65 % et de préférence au moins 70 % du poids total de la composition.

Par ailleurs, la phase aqueuse de l'émulsion peut contenir un ou plusieurs alcools inférieurs tels que l'éthanol en une quantité allant de préférence jusqu'à 15 % et mieux jusqu'à 10 % du poids total de la composition, un ou plusieurs polyols tels que la glycérine et le propylène glycol en une quantité allant par exemple jusqu'à 20% et mieux jusqu'à 10 % du poids total de la composition.

Par ailleurs, la composition de l'invention contient comme seul agent émulsionnant le diméthicone copolyol de formule (I). Ce diméthicone copolyol peut se présenter sous forme d'un mélange avec une huile de silicone volatile ou non volatile, et notamment avec les cyclométhicones (D4 ou D5) et/ou les polydiméthylsiloxanes de différentes viscosités et notamment 5 cst et 10 cst.

On peut utiliser notamment dans la composition selon l'invention les mélanges suivants commercialisés par la société Dow Corning :
- mélange de composé de formule (I), de tétracyclométhicone (D4) et d'eau (rapport pondéral 10/88/2), commercialisé sous la dénomination DC 3225C ;
- mélange de composé de formule (I), de pentacyclométhicone (D5) et d'eau (rapport pondéral 10/88/2), commercialisé sous la dénomination DC 5225C ;
- mélange de composé de formule (I) et de polydiméthylsiloxane 5 cst (rapport pondéral 10/90), commercialisé sous la dénomination DC 3225C in 200 Fluid 5 cst ;
- mélange de composé de formule (I) et de polydiméthylsiloxane 10 cst (rapport pondéral 10/90), commercialisé selon sous la dénomination DC 3225C in 200 Fluid 10 cst ;
- mélange de composé de formule (I) et de pentacyclométhicone (D5) (rapport pondéral 43/57), commercialisé sous la dénomination DC 5185C.

L'agent émulsionnant de formule (I) est présent de préférence en une quantité en matière active allant de 0,5 à 5 % et mieux de 0,6 à 2 % en poids par rapport au poids total de la composition.

Bien que la composition soit exempte de tout autre agent émulsionnant, la composition obtenue est stable dans le temps.

De préférence, le rapport pondéral phase huileuse/agent émulsionnant est égal ou supérieur à 5 et mieux égal ou supérieur à 8.

La phase huileuse de la composition selon l'invention peut renfermer, outre l'huile de silicone éventuellement en mélange avec l'agent émulsionnant, toute sorte d'huiles et de corps gras bien connus de l'homme du métier, comme les huiles d'origine végétale, les huiles d'origine animale, les huiles de synthèse et notamment les esters gras, les huiles de silicone, les huiles fluorées et/ou les huiles minérales, ainsi que les mélanges de ces huiles.

De préférence, la phase huileuse de la composition de l'invention comprend au moins une huile de silicone volatile généralement présente en une quantité allant de 6 à 16 % en poids par rapport au poids total de l'émulsion comme, par exemple, une silicone cyclique telle que la pentacyclométhicone, la tétracyclométhicone ou l'hexacyclométhicone.

La phase huileuse peut contenir, en outre d'autres constituants gras tels que les alcools gras et les acides gras.

La phase huileuse est présente dans la composition selon l'invention en une quantité allant de 8 à 22 % et de préférence de 12 à 20 % en poids par rapport au poids total de la composition.

Un autre avantage de la composition selon l'invention provient de ce qu'on peut y incorporer une grande quantité d'électrolyte sans nuire à la stabilité de la composition.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'a-hydroxyacides (citrates, tartrates, lactates, malates) ou d'acides de fruits, ou encore les sels d'acides aminés (aspartate, arginate, glycocholate, fumarate).

De préférence, l'électrolyte est un mélange de sels comprenant notamment des sels de calcium, de magnésium, et de sodium, et notamment un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte, lorsqu'il est présent, va en général de 0,5 à 20 % et de préférence de 2,5 à 10 % en poids par rapport au poids total de la composition.

De préférence, la composition selon l'invention est destinée à un soin ou un traitement topique. Dans ce cas, l'émulsion doit contenir un milieu physiologiquement acceptable, c'est-à-dire compatible avec la peau, les muqueuses, et/ou le cuir chevelu . En outre, elle contient de préférence au moins un actif et trouve son application dans un grand nombre de traitements cosmétiques et/ou dermatologiques de la peau, y compris du cuir chevelu, et/ou des muqueuses. en particulier pour le soin et/ou le maquillage et/ou la protection solaire de la peau et/ou des muqueuses, ainsi que pour la préparation d'une crème destinée au traitement des maladies de la peau, plus particulièrement de la peau grasse (apport de fraîcheur) et du psoriasis en tant que produit d'accompagnement du traitement.

L'invention a donc aussi pour objet une composition topique, caractérisée en ce qu'elle contient une émulsion telle que définie ci-dessus et au moins un actif.

Comme actifs, on peut citer notamment, outre les électrolytes indiqués ci-dessus, les polyols tels que la glycérine, les glycols comme le Polyéthylène glycol 8, et les dérivés de sucre, les enzymes, les extraits naturels, les oligomères procyannidoliques, les vitamines telles que la vitamine C, la vitamine E, la vitamine A et leurs esters, les dérivés phosphatés et glucosylés, l'urée, la rutine, les dépigmentants tels que l'acide kojique et l'acide caféique, les bêta-hydroxyacides tels que l'acide salicylique et ses dérivés, les alpha-hydroxyacides tels que l'acide lactique et l'acide glycolique, l'acide rétinoique et ses dérivés, les filtres, les hydratants tels que les hydrolysats de protéines et leurs mélanges.

L'actif peut être par exemple présent en une concentration allant de 0,01 à 20 %, de préférence de 0,1 à 5 % et mieux de 0,5 à 3 % du poids total de la composition.

De façon connue, la composition de l'invention peut contenir également des adjuvants habituels dans les domaines cosmétique et/ou dermatologique, tels que les conservateurs, les antioxydants, les agents complexants, les solvants, les parfums, les charges, les filtres, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

La présente invention a encore pour objet un procédé de traitement cosmétique de la peau, du cuir chevelu et/ou des muqueuses, caractérisé par le fait que l'on applique sur la peau, le cuir chevelu et/ou les muqueuses, une composition telle que définie ci-dessus.

L'invention a aussi pour objet l'utilisation de la composition telle que définie ci-dessus pour la fabrication d'une crème destinée au traitement des peaux grasses.

Quand elle contient des sels de la mer morte, la composition convient notamment pour le traitement du psoriasis. Aussi, la présente invention a aussi pour objet l'utilisation d'une telle composition pour la fabrication d'une crème destinée au traitement du psoriasis.

Les exemples ci-après de compositions selon l'invention sont donnés à titre d'illustration. Les quantités y sont données en % en poids.

### Exemple 1 : crème pour le visage

### A. Phase huileuse

- Diméthicone copolyol de formule (I) dans pentacyclométhicone et eau (10/88/2) (DC 5225 C) 17,5 %

### B. Phase aqueuse

- Chlorure de sodium 2,5 %
- Glycérine 5 %
- Conservateurs 0,55 %
- Acide citrique 0,035 %
- Eau 74,415 %

Mode opératoire : on prépare la phase B en chauffant le mélange du chlorure de sodium, de la glycérine et des conservateurs dans de l'eau, jusqu'à 45°C sous agitation jusqu'à dissolution complète des conservateurs. On laisse refroidir jusqu'à température ambiante, puis on y ajoute l'acide citrique dissout dans l'eau.
On prépare la phase A en mélangeant les constituants sous agitation et on verse le mélange précédemment obtenu dans la phase A sous agitation.

On obtient une crème blanche ayant une viscosité mesurée au RHEOMAT 180, de 9,94 Pa.s (99,4 poises) au temps zéro. Cette viscosité se stabilise après 10 minutes à 7,01 Pa.s (70,1 poises).

### Exemple 2 : crème pour le corps

### A. Phase huileuse

- Diméthicone copolyol de formule (I) dans tétracyclométhicone et eau (10/88/2) (DC 3225 C) 6,25 %
- Tétracyclométhicone 6,25 %

### B. Phase aqueuse

- Chlorure de sodium 2,5 %
- Polyéthylène glycol 8 2 %
- Eau 83 %

Mode opératoire : on prépare les différentes phases et on introduit la phase B dans la phase A sous agitation.

On obtient une crème blanche ayant une viscosité mesurée au RHEOMAT 180, de 4,45 Pa.s (44,5 poises), au temps zéro. Cette viscosité se stabilise après 10 minutes à 3,88 Pa.s (38,8 poises).

## Revendications

1. Composition comprenant une phase aqueuse dispersée dans une phase huileuse à l'aide d'un agent émulsionnant siliconé, **caractérisée par le fait qu'**elle a une viscosité mesurée au viscosimètre RHEOMAT 180 à un taux de cisaillement de 200 s⁻¹ et à 25 °C, allant de 3 Pa.s (30 poises) à 20 Pa.s (200 poises), qu'elle comporte au moins 78 % en poids de phase aqueuse par rapport au poids total de la composition dont au moins 65 % d'eau par rapport au poids total de la composition, et qu'elle contient comme seul agent émulsionnant, le diméthicone copolyol de formule (I) : 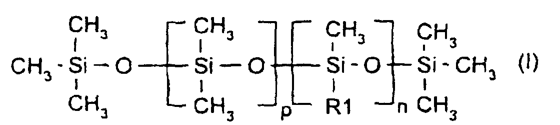 dans laquelle R1 représente -(C₃H₆O)-(OC₂H₄)₁₈-(C₃H₆O)₁₈H, p=394 et n=4.

2. Composition selon la revendication 1, **caractérisée par le fait que** l'agent émulsionnant est en mélange avec au moins une huile de silicone.

3. Composition selon la revendication 1 ou 2, **caractérisée par le fait que** l'agent émulsionnant est présent en une quantité allant de 0,5 à 5 % en poids par rapport au poids total de la. composition.

4. Composition selon l'une quelconque des revendications précédentes, **caractérisée par le fait que** la phase huileuse est présente en une quantité allant de 8 à 22 % en poids par rapport au poids total de la composition.

5. Composition selon l'une quelconque des revendications précédentes, **caractérisée par le fait que** le rapport pondéral phase huileuse/agent émulsionnant est égal ou supérieur à 5.

6. Composition selon l'une quelconque des revendications précédentes, **caractérisée par le fait qu'**elle contient au moins un électrolyte.

7. Composition selon la revendication précédente, **caractérisée par le fait que** l'électrolyte est présent en une quantité allant de 0,5 à 20 % du poids total de la composition.

8. Composition selon l'une quelconque des revendications précédentes, **caractérisée par le fait qu'**elle contient un milieu physiologiquement acceptable et constitue une composition topique.

9. Composition selon l'une quelconque des revendications précédentes, **caractérisée par le fait qu'**elle contient au moins un actif choisi parmi les polyols, les enzymes, les extraits naturels, les oligomères procyannidoliques, les vitamines, les dérivés phosphatés et glucosylés, l'urée, la rutine, les dépigmentants, les bêta-hydroxyacides, les alpha-hydroxyacides, l'acide rétinoique et ses dérivés, les filtres, les hydratants et leurs mélanges.

10. Composition selon l'une quelconque des revendications précédentes, **caractérisée par le fait qu'**elle contient un mélange de sels de la mer morte.

11. Procédé de traitement cosmétique de la peau, du cuir chevelu et/ou des muqueuses, **caractérisé par le fait que** l'on applique sur la peau, le cuir chevelu et/ou les muqueuses, une composition selon l'une quelconque des revendications 1 à 10.

12. Utilisation de la composition selon l'une quelconque des revendications 1 à 10 pour la fabrication d'une crème destinée au traitement des peaux grasses.

13. Utilisation de la composition selon la revendication 10 pour la fabrication d'une crème destinée au traitement du psoriasis.

## Patentansprüche

1. Zusammensetzung, die eine wässrige Phase enthält, die mit Hilfe eines siliconhaltigen Emulgators in einer Ölphase dispergiert ist, **dadurch gekennzeichnet, dass** sie eine mit einem Viskosimeter RHEOMAT 180 bei einer Schergeschwindigkeit von 200 s⁻¹ und bei 25 °C gemessene Viskosität im Bereich von 3 Pa·s (30 Poise) bis 20 Pa·s (200 Poise) aufweist, dass sie mindestens 78 Gew.-% wässrige Phase, bezogen auf das Gesamtgewicht der Zusammensetzung enthält, wobei die wässrige Phase mindestens 65 % Wasser enthält, bezogen auf das Gesamtgewicht der Zusammensetzung, und dass sie als einzigen Emulgator den Dimeticon-Copolyol der Formel (I) 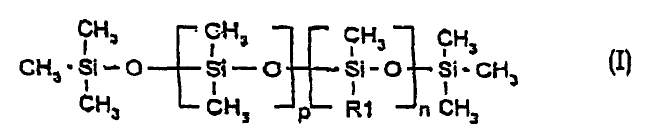 enthält, in der R1 -(C₃H₆O)-(OC₂H₄)₁₈-(C₃H₆O)₁₈H, p = 394 und n = 4 bedeutet.

2. Zusammensetzung nach Anspruch 1, **dadurch gekennzeichnet, dass** der Emulgator im Gemisch mit mindestens einem Siliconöl vorliegt.

3. Zusammensetzung nach Anspruch 1 oder 2, **dadurch gekennzeichnet, dass** der Emulgator der Formel (I) in einem Mengenanteil im Bereich von 0,5 bis 5 Gew.-%, bezogen auf das Gesamtgewicht der Zusammensetzung, enthalten ist.

4. Zusammensetzung nach einem der vorhergehenden Ansprüche, **dadurch gekennzeichnet, dass** die Ölphase in einem Mengenanteil von 8 bis 22 Gew.-%, bezogen auf das Gesamtgewicht der Zusammensetzung, enthalten ist.

5. Zusammensetzung nach einem der vorhergehenden Ansprüche, **dadurch gekennzeichnet, dass** das Gewichtsverhältnis Ölphase/Emulgator mindestens 5 beträgt.

6. Zusammensetzung nach einem der vorhergehenden Ansprüche, **dadurch gekennzeichnet, dass** sie mindestens einen Elektrolyten enthält.

7. Zusammensetzung nach dem vorhergehenden Anspruch, **dadurch gekennzeichnet, dass** der Elektrolyt in einem Mengenanteil von 0,5 bis 20 % des Gesamtgewichts der Zusammensetzung enthalten ist.

8. Zusammensetzung nach einem der vorhergehenden Ansprüche, **dadurch gekennzeichnet, dass** sie ein physiologisch akzeptables Medium enthält und eine topische Zusammensetzung bildet.

9. Zusammensetzung nach einem der vorhergehenden Ansprüche, **dadurch gekennzeichnet, dass** sie mindestens einen Wirkstoff enthält, der unter Polyolen, Enzymen, natürlichen Extrakten, Procyanidin-Oligomeren, Vitaminen, phosphathaltigen und glykosylierten Derivaten, Harnstoff, Rutin, Depigmentierungsmitteln, β-Hydroxysäuren, α-Hydroxysäuren, Retinoesäure und ihren Derivaten, Filtern, Hydratisierungsmitteln und ihren Gemischen ausgewählt ist.

10. Zusammensetzung nach einem der vorhergehenden Ansprüche, **dadurch gekennzeichnet, dass** sie ein Gemisch von Salzen aus dem Toten Meer enthält.

11. Verfahren zur kosmetischen Behandlung der Haut, der Kopfhaut und/oder der Schleimhäute, **dadurch gekennzeichnet, dass** auf die Haut, die Kopfhaut und/oder die Schleimhäute eine Zusammensetzung nach einem der Ansprüche 1 bis 10 aufgetragen wird.

12. Verwendung der Zusammensetzung nach einem der Ansprüche 1 bis 10 für die Herstellung einer Creme, die für die Behandlung von fettiger Haut vorgesehen ist.

13. Verwendung der Zusammensetzung nach einem der Ansprüche 1 bis 10 für die Herstellung einer Creme, die für die Behandlung der Psoriasis vorgesehen ist.

## Claims

1. Composition comprising an aqueous phase dispersed in an oily phase using a silicone emulsifier, **characterized in that** it has a viscosity, measured using a Rheomat 180 viscometer at a shear rate of 200 s⁻¹ and at 25°C, ranging from 3 Pa.s (30 poises) to 20 Pa.s (200 poises), **in that** it comprises at least 78% by weight of aqueous phase relative to the total weight of the composition, including at least 65% water relative to the total weight of the composition, and **in that** it contains as sole emulsifier the dimethicone copolyol of formula (I): 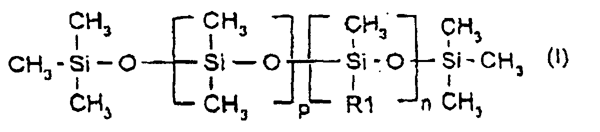 in which R1 represents -(C₃H₆O)-(OC₂H₄)₁₈-(C₃H₆O)₁₈H, p = 394 and n = 4.

2. Composition according to Claim 1, **characterized in that** the emulsifier is in a mixture with at least one silicone oil.

3. Composition according to Claim 1 or 2, **characterized in that** the emulsifier is present in an amount ranging from 0.5% to 5% by weight relative to the total weight of the composition.

4. Composition according to any one of the preceding claims, **characterized in that** the oily phase is present in an amount ranging from 8% to 22% by weight relative to the total weight of the composition.

5. Composition according to any one of the preceding claims, **characterized in that** the oily phase/emulsifier weight ratio is greater than or equal to 5.

6. Composition according to any one of the preceding claims, **characterized in that** it contains at least one electrolyte.

7. Composition according to the preceding claim, **characterized in that** the electrolyte is present in an amount ranging from 0.5% to 20% relative to the total weight of the composition.

8. Composition according to any one of the preceding claims, **characterized in that** it contains a physiologically acceptable medium and constitutes a topical composition.

9. Composition according to any one of the preceding claims, **characterized in that** it contains at least one active agent chosen from polyols, enzymes, natural extracts, procyanidol oligomers, vitamins, glucosyl and phosphate derivatives, urea, rutin, depigmenting agents, beta-hydroxy acids, alpha-hydroxy acids, retinoic acid and its derivatives, screening agents, and moisturizers, and mixtures thereof.

10. Composition according to any one of the preceding claims, **characterized in that** it contains a mixture of Dead Sea salts.

11. Cosmetic process for treating the skin, the scalp and/or mucous membranes, **characterized in that** a composition according to any one of Claims 1 to 10 is applied to the skin, the scalp and/or mucous membranes.

12. Use of the composition according to any one of Claims 1 to 10 for the manufacture of a cream intended for treating greasy skin.

13. Use of the composition according to Claim 10 for the manufacture of a cream intended for treating psoriasis.
